# FASCICULE DE BREVET EUROPEEN

(11) **EP 1 297 793 B1**
(45) Date de publication et mention de la délivrance du brevet: **24.11.2004**
(21) Numéro de dépôt: 02356182.2
(22) Date de dépôt: 24.09.2002
(51) Int. Cl.: A61B 17/80

(54) **Plaque de fixation des os d'une articulation, en particulier d'une articulation métatarso-phalangienne**
Befestigungsplatte für Knochen eines Gelenks, insbesondere für ein Metatarsophalangealgelenk
Fixation plate for joint bones, in particular for a metatarso-phalangeal joint

(30) Priorité: 26.09.2001 FR 0112417
(43) Date de publication de la demande: 02.04.2003
(73) Titulaire: Newdeal S.A., 69006 Lyon (FR)
(72) Inventeur: Coughlin, Michael John, Boise, Idaho (US)
(74) Mandataire: Martin, Didier Roland Valéry

(56) Documents cités:
- EP-A- 1 132 052
- US-A- 4 800 874
- US-A- 5 853 413
- US-B1- 6 221 073

## Description

La présente invention se rapporte au domaine technique général des dispositifs chirurgicaux destinés à assurer la solidarisation et l'alignement relatifs de deux parties osseuses d'une articulation, et en particulier d'une articulation comportant des phalanges d'orteil ou de doigt en vue de réaliser une arthrodèse.

Le document US-A-5,853,413 décrit une plaque de fixation d'os destinée à une arthrodèse du poignet, ladite plaque comprenant deux sections, respectivement proximale et distale, présentant chacune un axe de symétrie longitudinal, respectivement S₁, S₂, ladite plaque étant destinée à être déposée sur l'articulation.

La présente invention concerne une plaque de fixation des os d'une articulation, en particulier d'une articulation métatarso-phalangienne, destinée à réaliser une arthrodèse.

Dans une application préférentielle, mais non exclusive de l'invention, la plaque de fixation sera plus particulièrement, mais non exclusivement, destinée et conçue pour assurer le positionnement angulaire puis l'arthrodèse d'une articulation métatarso-phalangienne, étant entendu néanmoins au sens de l'invention que des applications à d'autres articulations peuvent être envisagées dès l'instant où il s'agit d'assurer la solidarisation relative entre deux parties osseuses de l'articulation.

Dans le cas d'arthrose survenant au niveau des articulations osseuses, et en particulier au niveau de l'articulation métatarso-phalangienne, il s'avère souvent nécessaire de pratiquer une arthrodèse afin de fusionner les deux os entre eux. De manière générale, les arthrodèses sont des opérations délicates, puisqu'en définitive elles bloquent une articulation dans une position définie et de manière irréversible. C'est ainsi que l'arthrodèse de l'articulation métatarso-phalangienne revêt un caractère hautement important, dans la mesure où cette articulation intervient de manière essentielle dans le cycle de la marche d'un être humain. On comprend alors qu'il s'avère essentiel que l'orientation entre les deux os, au moment de leur positionnement relatif avant la fusion, soit réalisée avec la meilleure précision possible pour éviter toute gêne ultérieure.

Il s'avère donc essentiel de fixer les axes relatifs des os pour respecter au mieux les flexions et axes du patient, et ce en fonction du sexe du patient, de sa démarche et de sa morphologie.

Jusqu'à présent, les arthrodèses métatarso-phalangiennes sont réalisées à l'aide de plaques de fixation de formes diverses et pourvues de lumières destinées à recevoir des vis de fixation pour assurer la solidarisation de la plaque avec les deux os à fusionner.

Ainsi, on connaît par exemple des plaques de fixation présentant une section transversale courbe dite *« en quart de tubes »* que le chirurgien met en place à cheval sur l'articulation entre les deux os à fusionner. Cette plaque connue est cintrée par le chirurgien lui-même dans un plan correspondant à l'angle de dorsi-flexion selon une valeur déterminée par le chirurgien et propre au patient. Les plaques de ce type permettent donc de conférer à l'articulation, lorsqu'elle est bloquée, une orientation en élévation de l'un des deux os lorsque l'arthrodèse est réalisée. Ceci constitue un avantage pour le patient puisque l'articulation bloquée se rapproche de conditions normales d'utilisation de l'articulation, ce qui réduit la gêne du patient lors de la marche et réduit d'éventuelles complications futures. Néanmoins, les plaques de fixation de ce type s'avèrent souffrir de divers inconvénients, et notamment d'une certaine difficulté lors de la mise en oeuvre du cintrage par le chirurgien. En outre, il s'avère d'une part que le cintrage est rarement réalisé avec suffisamment de précision et, d'autre part, que cette opération fait apparaître des zones déformées de la plaque présentant des arêtes plus ou moins vives, susceptibles de générer des irritations voire des inflammations lorsqu'elles sont en contact avec les tissus environnants tels que tendons, muscles, ligaments, peau, etc. Enfin, les plaques de ce type ne permettent pas un cintrage selon l'angle en *varus-valgus*, de telle sorte qu'elles ne permettent pas de réaliser des arthrodèses suffisamment proches des conditions optimales d'orientation géométrique entre les deux os à fusionner, pour réduire de la meilleure façon possible les risques de gênes et complications ultérieures pour le patient.

On connaît également des plaques de fixation entièrement planes qui, si elles réduisent fortement tout risque de complications ultérieures pour le patient consécutivement à l'opération de mise en place dans la mesure où aucune opération de cintrage n'intervient, ne permettent néanmoins pas de réaliser une arthrodèse dont les os présentent un angle de dorsi-flexion et un angle de *varus-valgus* particuliers.

Il s'avère également, de manière générale, que les plaques de fixation destinées à être cintrées par le chirurgien nécessitent le recours à un outil spécifique de cintrage adapté à chaque type de plaque, ce qui constitue une contrainte supplémentaire. Enfin, il s'avère que les plaques de fixation cintrées induisent une fragilisation du métal au niveau de la zone de cintrage, ce qui présente un inconvénient en terme de résistance mécanique. De plus, la nécessité de prévoir des ouvertures pour le passage de vis de fixation dans la plaque complique l'opération de cintrage de la plaque en raison précisément de la présence de ces ouvertures.

L'objet de l'invention vise en conséquence à porter remède aux différents inconvénients énumérés précédemment et à proposer une nouvelle plaque de fixation des os d'une articulation, en particulier d'une articulation métatarso-phalangienne, destinée à réaliser une arthrodèse entre deux fragments osseux dans des conditions optimales tout en permettant une excellente précision de l'orientation relative des deux os à fusionner.

Un autre objet de l'invention vise à proposer une nouvelle plaque de fixation des os d'une articulation facilitant la mise en place de la plaque et la recherche d'une orientation géométrique précise.

Un autre objet de l'invention est de proposer une nouvelle plaque de fixation des os d'une articulation dont la mise en place est améliorée.

Un autre objet de l'invention vise à proposer une nouvelle plaque de fixation des os d'une articulation particulièrement bien adaptée à l'anatomie des os à fusionner.

Un autre objet de l'invention vise à proposer une nouvelle plaque de fixation des os d'une articulation susceptible de s'adapter à différentes configurations anatomiques possibles.

Les objets assignés à l'invention sont atteints à l'aide d'une plaque de fixation des os d'une articulation, en particulier d'une articulation métatarso-phalangienne, destinée à réaliser une arthrodèse, caractérisée en ce que :
- la plaque est composée de deux sections, respectivement proximale et distale, présentant chacune un axe de symétrie longitudinal, respectivement S₁, S₂, ladite plaque étant destinée à être déposée à cheval sur l'articulation, les axes S₁, S₂ étant parallèles et alignés avec les axes longitudinaux des deux fragments osseux à fusionner, de telle sorte que selon un plan horizontal, la projection de l'axe de symétrie S₂ de la section distale présente une inclinaison angulaire α par rapport à la projection de l'axe de symétrie S₁ de la partie proximale, leur intersection se faisant au point A₁,
- la projection selon un plan vertical de l'axe de symétrie S₂ présente une inclinaison (3 par rapport à la projection de l'axe de symétrie S₁, leur intersection se faisant au point A₂ qui est distinct du point A₁, la section distale s'étendant sur une première portion de longueur L₁ à partir de la section proximale dans le même plan d'extension P₁ de la section proximale.

D'autres avantages et objets de l'invention apparaîtront plus en détails à la lecture de la description qui suit, et à l'aide des dessins annexés fournis à titre purement explicatif et non limitatif, dans lesquels :
- La figure 1 illustre, selon une vue schématique en perspective, une plaque de fixation des os d'une articulation mise en place sur une articulation métatarso-phalangienne en vue de réaliser une arthrodèse.
- La figure 2 illustre, selon une vue de dessus correspondant à un plan horizontal, une plaque de fixation des os d'une articulation conforme à l'invention présentant une inclinaison sur la gauche.
- La figure 3 illustre, selon une vue latérale partielle correspondant à un plan vertical, une plaque de fixation des os d'une articulation conforme à l'invention.

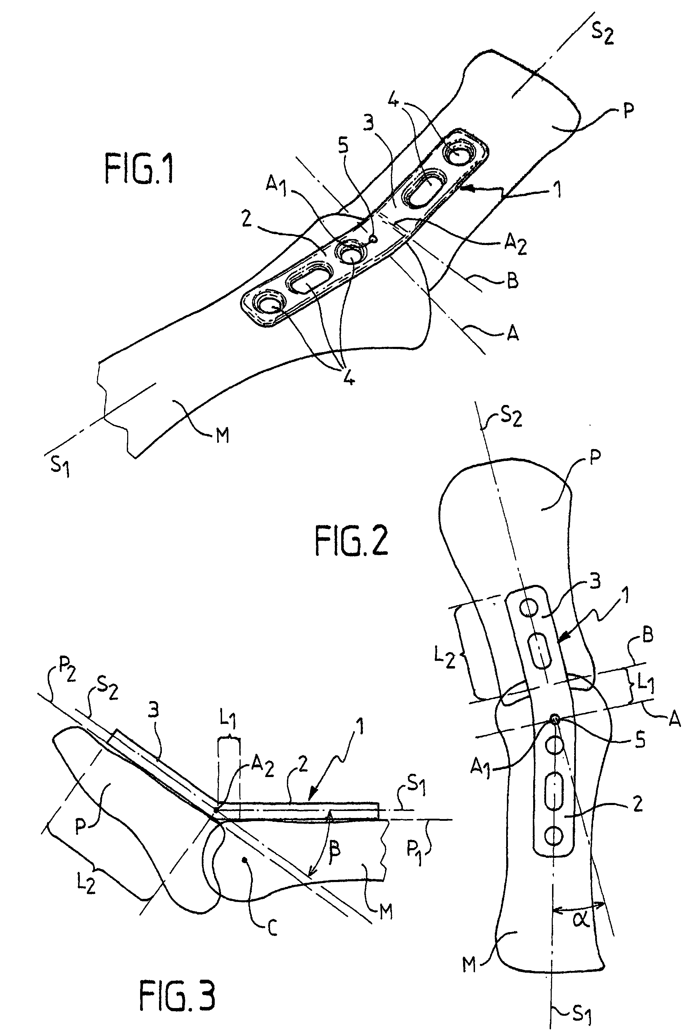

Les figures 1 à 3 montrent une plaque de fixation 1 mise en place et fixée sur une articulation métatarso-phalangienne en vue de réaliser une arthrodèse entre le métatarsien M et la phalange P.

Néanmoins, au sens de l'invention, la plaque de fixation 1 conforme à l'invention pourra être utilisée moyennant une conformation et un dimensionnement anatomique appropriés, pour réaliser des arthrodèses d'autres articulations, en particulier pour des articulations de la main dans le cadre d'opérations de chirurgie de la main.

La plaque de fixation 1 conforme à l'invention se présente sous la forme d'un corps allongé, de section transversale par exemple plane, réalisé par exemple en métal, et de rigidité suffisante pour assurer une jonction entre les os de l'articulation à fusionner et assurer un support ferme.

Selon l'invention la plaque de fixation 1 est composée de deux sections 2, 3, respectivement proximale et distale, présentant chacune un axe de symétrie longitudinal, respectivement S₁, S₂.

Chaque section 2, 3 peut donc être assimilée de manière générale à un corps d'une épaisseur de quelques millimètres et d'une longueur L supérieure à sa largeur ℓ, de manière à former une forme géométrique généralement assimilable à un parallélépipède rectangle.

La section proximale 2 est destinée à être mise en place et fixée sensiblement sur le métatarsien M, alors que la section distale 3 est destinée à être mise en place et fixée sensiblement sur la phalange P en vue de réaliser l'arthrodèse entre ces deux fragments osseux, la plaque 1 étant donc disposée à cheval sur l'articulation, les axes de symétrie S₁, S₂ étant parallèles et alignés avec les axes de symétrie longitudinaux des os.

De manière connue, la fixation intervient par l'intermédiaire de vis (non représentées aux figures) qui sont vissées par le chirurgien dans les os à travers une série de lumières 4 ménagées dans la plaque de fixation 1, et en particulier dans chacune des sections 2, 3 pour permettre le passage des vis de fixation.

Tel qu'illustré aux figures, la section proximale 2 est avantageusement de longueur supérieure à la section distale 3, tout en étant d'épaisseur égale et de largeur également égale. En raison de la longueur supérieure de la section proximale 2, cette dernière comportera avantageusement un nombre supérieur d'orifices 4, et par exemple trois orifices, alors que la section distale 3 comportera deux orifices 4, ces derniers étant de forme quelconque, et par exemple circulaire ou oblongue.

Les sections distale 3 et proximale 2 sont avantageusement de longueur identique. Dans tous les cas, les sections distale 3 et proximale 2 sont adjacentes et jointives.

Cette particularité dimensionnelle permet une meilleure adaptation anatomique de la plaque de fixation aux os à fusionner et améliore la capacité de support et de maintien de ladite plaque.

Avantageusement, les deux sections 2, 3 seront planes et rectilignes, étant entendu qu'à titre de variante, les sections transversales de la section proximale 2 et de la section distale 3 pourront présenter une certaine courbure, de préférence égale, pour s'adapter au mieux à certaines configurations anatomiques particulières.

Selon une particularité importante de l'invention, tel qu'illustré en particulier à la figure 2, les axes de symétrie S₁, S₂ ne sont pas alignés l'un par rapport à l'autre mais présentent au contraire une certaine inclinaison angulaire, de telle manière que les deux sections présentent une inclinaison respective dans le plan horizontal. Ainsi, selon l'invention, la projection de l'axe de symétrie S₂ de la section distale 3 sur un plan horizontal présente une inclinaison angulaire α par rapport à la projection de l'axe de symétrie S₁ de la partie proximale 2, leur intersection se faisant au point A₁.

Cette particularité permet de conférer un angle de *varus-valgus* précis et pré-établi à la phalange relativement au métatarse, les axes de symétrie S₁, S₂ de la plaque 1 étant destinés lors du montage par un chirurgien à être strictement parallèles aux axes longitudinaux des deux fragments osseux à fusionner M, P.

Selon l'invention, l'inclinaison angulaire α sera comprise entre 5° et 20° et préférentiellement elle sera de l'ordre de 10°. Il est précisé que l'inclinaison angulaire α entre les sections proximale 2 et distale 3 est une valeur angulaire fixe et donnée de la plaque de fixation 1 conforme à l'invention, évitant au chirurgien d'avoir à effectuer un cintrage de la plaque pendant l'opération, comme c'est le cas dans l'art antérieur connu. La section distale 3 peut être indifféremment inclinée sur la gauche ou sur la droite par rapport à la section proximale 2.

Selon une autre caractéristique importante de l'invention, la plaque de fixation 1 est telle que la projection selon un plan vertical (Figure 3) de l'axe de symétrie S₂ présente une inclinaison β par rapport à la projection de l'axe de symétrie S₁, leur intersection se faisant au point A₂ qui est distinct du point A₁.

Selon cette caractéristique importante de l'invention, tel qu'illustré en particulier aux figures 1 et 3, la section distale 3 s'étend sur une première portion de longueur L₁ à partir de la section proximale 2 dans le même plan d'extension P₁ de la section proximale 2, ladite première portion de longueur L₁ se prolongeant par une seconde portion de longueur L₂ qui s'étend dans un plan P₂ formant un angle avec le plan d'extension P₁, en l'occurrence l'angle β. L'angle β permet de conférer à la phalange P un angle de dorsi-flexion précis et pré-établi.

Selon cette caractéristique, la somme des longueurs L₁ et L₂ correspond à la longueur totale de la section distale 3 débutant à la ligne d'inclinaison A (figure 1), marquant le changement de direction entre les deux sections 2, 3. Ainsi, les section proximale 2 et distale 3 sont liées entre elles directement sans zone de liaison, et tangentes à l'unique rayon de courbure formant leur liaison.

Selon cette caractéristique, il apparaît que la plaque de fixation conforme à l'invention comporte deux inclinaisons distinctes, l'une dans le plan horizontal correspondant à la valeur angulaire α responsable de l'angle en *varus-valgus* et à la ligne A, l'autre correspondant à l'angle β responsable de l'angle de dorsi-flexion et à la ligne B, les deux zones d'inclinaison de la plaque n'étant pas confondues mais au contraire décalées l'une par rapport à l'autre, la zone de changement d'inclinaison B étant située en position distale relativement à la ligne A.

Avantageusement l'angle β est compris entre 5° et 20°, et il est de préférence de l'ordre de 10°. Cet angle β est modifiable par le chirurgien préalablement ou en cours de l'intervention chirurgicale.

Cette disposition permet d'obtenir une meilleure congruence entre la plaque de fixation 1 et l'ensemble des parties osseuses à fusionner, puisque seule la partie terminale L₂, de la section distale 3 présente une double inclinaison à la fois dans le plan horizontal et dans le plan vertical, alors que l'autre fraction de la section distale ne présente qu'une seule inclinaison dans le plan horizontal.

Selon une caractéristique particulièrement avantageuse de l'invention, la plaque de fixation 1 sera pourvue d'un orifice 5 de fixation intermédiaire. Avantageusement, l'orifice 5 de fixation intermédiaire est situé à l'intersection des axes de symétrie S₁ et S₂, c'est-à-dire sur la ligne d'inclinaison A, et correspond au point A₁. Cet orifice permet au chirurgien de fixer de façon provisoire la plaque de fixation 1 à l'aplomb du centre C de la tête du métatarse M par l'intermédiaire d'une broche, pour ensuite mettre en place les vis de fixation définitives dans les lumières 4.

Le mode de mise en place de la plaque de fixation conforme à l'invention est le suivant: après avoir réalisé une incision au niveau de l'articulation à fusionner, le praticien réalise soit un fraisage ou encore une coupe des faces articulaires afin de supprimer l'arthrose présente dans l'articulation. Par la suite, le chirurgien met en place les deux os à fusionner l'un par rapport l'autre, puis applique la plaque de fixation 1 conforme à l'invention sur les faces supérieures des os M et P.

Cette application doit être réalisée en veillant à positionner la plaque de fixation 1 de telle manière que les axes de symétrie S₁, S₂ sont strictement parallèles et alignés avec les axes longitudinaux des deux fragments osseux m, P à fusionner. Par ailleurs, le chirurgien doit veiller à positionner la section proximale 2, de telle manière que l'orifice 5 de fixation intermédiaire soit sensiblement au droit du centre C de la tête du métatarse M pour que la congruence entre la plaque et les parties osseuses soit optimale. La fixation proprement dite des os dans la configuration géométrique requise s'effectue ensuite naturellement en raison des deux inclinaisons naturelles fixes et préétablies de la plaque de fixation conforme à l'invention.

La plaque de fixation conforme à l'invention permet donc de réaliser de manière simple, rapide et précise une arthrodèse des os d'une articulation selon une configuration géométrique précise et pré-établie.

## Revendications

1. Plaque de fixation (1) des os d'une articulation, en particulier d'une articulation métatarso-phalangienne, destinée à réaliser une arthrodèse,
- ladite plaque (1) étant composée de deux sections (2, 3), respectivement proximale (2) et distale (3), présentant chacune un axe de symétrie longitudinal, respectivement S₁, S₂, ladite plaque étant destinée à être déposée à cheval sur l'articulation, les axes S₁, S₂ étant parallèles et alignés avec les axes longitudinaux des deux fragments osseux à fusionner, de telle sorte que selon un plan horizontal, la projection de l'axe de symétrie S₂ de la section distale (3) présente une inclinaison angulaire α par rapport à la projection de l'axe de symétrie S₁ de la partie proximale (2), leur intersection se faisant au point A₁,
- la projection selon un plan vertical de l'axe de symétrie S₂ présentant une inclinaison β par rapport à la projection de l'axe de symétrie S₁, leur intersection se faisant au point A₂ qui est distinct du point A₁, la section distale (3) s'étendant sur une première portion de longueur L₁ à partir de la section proximale (2) dans le même plan d'extension P₁ de la section proximale (2).

2. Plaque selon la revendication 1 **caractérisée en ce qu'**elle est pourvue d'un orifice (5) de fixation intermédiaire pour le passage d'une vis de fixation, ledit orifice (5) étant situé à l'intersection des axes de symétrie S₁ et S₂.

3. Plaque selon la revendication 1 ou 2 **caractérisée en ce que** l'inclinaison angulaire α est comprise entre 5° et 20° et l'angle β est compris entre 5° et 20°.

4. Plaque selon l'une des revendications 1 à 3 **caractérisée en ce que** l'inclinaison angulaire α est de l'ordre de 10° et l'angle β est de l'ordre de 10°.

5. Plaque selon l'une des revendications 1 à 4 **caractérisée en ce qu'**elle comporte des lumières (4) pour permettre le passage de vis de fixation.

6. Plaque selon l'une des revendications 1 à 5 **caractérisée en ce que** la section proximale (2) est de longueur supérieure à la longueur de la section distale (3).

7. Plaque selon l'une des revendications 1 à 5 **caractérisée en ce que** les sections distale (3) et proximale (2) sont de longueur identique.

8. Plaque selon l'une des revendications 1 à 7 **caractérisée en ce que** les sections distale (3) et proximale (2) sont planes.

9. Plaque selon l'une des revendications 1 à 8 **caractérisée en ce que** la section distale (3) est inclinée sur la gauche ou sur la droite par rapport à la section proximale (2).

## Patentansprüche

1. Befestigungsplatte (1) für Gelenkknochen, insbesondere für ein Metatarsophalangealgelenk, welche für die Durchführung einer Arthrodese bestimmt ist, wobei die Platte (1) sich aus zwei Abschnitten (2, 3), einem proximalen (2) bzw. distalen. (3), zusammensetzt, welche jeweils in Längsrichtung eine Symmetrieachse S₁ bzw. S₂ aufweisen, die Platte dafür bestimmt ist, auf das Gelenk aufgesetzt zu werden,
die Achsen S₁ und S₂ parallel zueinander und in gerader Linie zu den Längsachsen der beiden zu konsolidierenden Knochenfragmente verlaufen, so dass die Projektion der Symmetrieachse S₂ des distalen Abschnitts (3) auf eine horizontale Ebene in Bezug auf die Projektion der Symmetrieachse S₁ des proximalen Abschnitts (2) einen Neigungswinkel α aufweist, wobei deren Schnittpunkt bei A, liegt,
die Projektion der Symmetrieachse S₂ auf eine vertikale Ebene in Bezug auf die Projektion der Symmetrieachse S₁ eine Neigung β aufweist, wobei deren Schnittpunkt bei A₂ liegt, der sich von Punkt A₁ unterscheidet, und
der distale Abschnitt (3) über einen ersten Längenteil L₁ vom proximalen Abschnitt (2) aus in derselben Ausbreitungsebene P₁ des proximalen Abschnitts (2) verläuft.

2. Platte nach Anspruch 1, **dadurch gekennzeichnet, dass** sie mit einer Zwischenöffnung (5) zur Befestigung versehen ist, damit eine Befestigungsschraube hindurch treten kann, wobei die Öffnung (5) am Schnittpunkt der Symmetrieachsen S₁ und S₂ angeordnet ist.

3. Platte nach Anspruch 1 oder 2, **dadurch gekennzeichnet, dass** der Neigungswinkel α zwischen 5° und 20° und der Winkel β zwischen 5° und 20° zu liegen kommt.

4. Platte nach den Ansprüchen 1 bis 3, **dadurch gekennzeichnet, dass** der Neigungswinkel α in der Größenordnung von 10° und der Winkel β in der Größenordnung von 10° liegt.

5. Platte nach einem der Ansprüche 1 bis 4, **dadurch gekennzeichnet, dass** sie Löcher (4) aufweist, damit Befestigungsschrauben hindurch treten können.

6. Platte nach einem der Ansprüche 1 bis 5, **dadurch gekennzeichnet, dass** der proximale Abschnitt (2) länger ist als der distale Abschnitt (3).

7. Platte nach einem der Ansprüche 1 bis 5, **dadurch gekennzeichnet, dass** der distale (3) und proximale Abschnitt (2) von gleicher Länge sind.

8. Platte nach einem der Ansprüche 1 bis 7, **dadurch gekennzeichnet, dass** der distale (3) und proximale Abschnitt (2) eben sind.

9. Platte nach einem der Ansprüche 1 bis 8, **dadurch gekennzeichnet, dass** der distale Abschnitt (3) in Bezug auf den proximalen Abschnitt (2) nach links oder rechts geneigt ist.

## Claims

1. Plate (1) for fixing bones of an articulation, in particular of a metatarsophalangeal articulation, intended to effect an arthrodesis,
- the said plate (1) being composed of two sections (2, 3), respectively proximal (2) and distal (3), each having a longitudinal symmetry axis, respectively S₁, S₂, the said plate being intended to be placed so as to straddle the articulation, the axes S₁, S₂ being parallel and aligned with the longitudinal axes of the two bone fragments to be fused, so that, in a horizontal plane, the projection of the axis of symmetry S₂ of the distal section (3) has an angular inclination α with respect to the projection of the symmetry axis Si of the proximal part (2), their intersection taking place at point A₁,
- the projection in a vertical plane of the symmetry axis S₂ having an inclination β with respect to the projection of the symmetry axis S₁, their intersection taking place at point A₂, which is distinct from point A₁, the distal section (3) extending over a first portion of length L₁ as from the proximal section (2) in the same extension plane P₁ of the proximal section (2).

2. Plate according to Claim 1, **characterised in that** it is provided with an intermediate fixing orifice (5) for the passage of a fixing screw, the said orifice (5) being situated at the intersection of the axes of symmetry S₁ and S₂.

3. Plate according to Claim 1 or 2, **characterised in that** the angular inclination α is between 5° and 20° and the angle β is between 5° and 20°.

4. Plate according to one of Claims 1 to 3, **characterised in that** the angular inclination α is around 10° and the angle β is around 10°.

5. Plate according to one of Claims 1 to 4, **characterised in that** it comprises apertures (4) to allow fixing screws to pass.

6. Plate according to one of Claims 1 to 5, **characterised in that** the proximal section (2) has a length greater than the length of the distal section (3).

7. Plate according to one of Claims 1 to 5, **characterised in that** the distal (3) and proximal (2) sections are identical in length.

8. Plate according to one of Claims 1 to 7, **characterised in that** the distal (3) and proximal (2) sections are flat.

9. Plate according to one of Claims 1 to 8, **characterised in that** the distal section (3) is inclined to the left or right with respect to the proximal section (2).
